(19) Europäisches Patentamt

European Patent Office

Office européen des brevets

(11) Veröffentlichungsnummer: **0 302 411 B1**

(12) **EUROPÄISCHE PATENTSCHRIFT**

(45) Veröffentlichungstag der Patentschrift: **17.06.92**

(21) Anmeldenummer: **88112363.2**

(22) Anmeldetag: **29.07.88**

(51) Int. Cl.⁵: **B05B 7/08,** A61M 11/06,
A61M 35/00, B05B 7/02

(54) **Sprühkopf zum Applizieren eines Mehrkomponentenmaterials mittels Gas.**

(30) Priorität: **01.08.87 DE 3725552**

(43) Veröffentlichungstag der Anmeldung:
**08.02.89 Patentblatt 89/06**

(45) Bekanntmachung des Hinweises auf die
Patenterteilung:
**17.06.92 Patentblatt 92/25**

(84) Benannte Vertragsstaaten:
**AT BE CH DE ES FR GB GR IT LI LU NL SE**

(56) Entgegenhaltungen:
**EP-A- 0 037 393**
**EP-A- 0 156 098**
**GB-A- 2 082 686**
**US-A- 4 361 285**

(73) Patentinhaber: **HOECHST AKTIENGESELL-
SCHAFT
Postfach 80 03 20
W-6230 Frankfurt am Main 80(DE)**

(72) Erfinder: **Zimmermann, Josef
Auf der Krautweide 11
W-6231 Sulzbach/Taunus(DE)**

## Beschreibung

Die Erfindung betrifft einen Sprühkopf zum Applizieren eines Mehrkomponentenmaterials, insbesondere zum Applizieren von chirurgischem Gewebeklebstoff.

Aus der europäischen Patentschrift 00 37 393 ist eine Vorrichtung zum Applizieren von chirurgischem Gewebeklebstoff bekannt. Die Spritzenkörper füur die Komponenten Fibrinogenlösung und Thrombinlösung sind über Konusse mit Föderkanälen eines Sprühkopfes verbunden. Der Sprühkopf weist einen Zuführkanal für Gas auf, der sich innerhalb des Sprühkopfes in zwei Äste teilt, der Austrittsöffnungen im Bereich der Mündungen der Förderkanäle angeordnet und im Winkel zueinander geführt sind und die rechtwinkelig zur Austrittsöffnung der Komponenten gerichtet sind. Die Komponenten, die über die Spritzenkörper (Kolbenpumpen) zugeführt werden, werden durch das Gas versprüht. Die Sprühkegel vereinigen sich je nach Größe des Winkels, den die Sprühachsen einschließen, mehr oder weniger entfernt vor den Mündungen der Förderkanäle, so sie sich durchmischen, und die Komponenten miteinander reagieren. Nachteilig ist, daß zielsicheres Sprühen mit diesem Sprühkopf nicht möglich ist, denn das zu besprühende Objekt muß genau im Schnittpunkt der Sprühstrahlen liegen, wenn ein befriedigendes Ergebnis erzielt werden soll.

Hier will die Erfindung Abhilfe schaffen. Gemäß der Erfindung, wie sie im Anspruch gekennzeichnet ist, wird die Aufgabe durch einen Sprühkopf gelöst, der einen Gaskanal mit Gasanschluß aufweist, der durch einen Trennsteg in mehrere, parallel zum Trennsteg verlaufende Kanäle aufgeteilt wird, in diese Kanäle Bohrungen zur Aufnahme von Kanülen Münden , deren offene Ende in Rinnen zum Führen der Kanülen übergehen, die parallel zum Gaskanal im Sprühkopf angeordnet sind und der Trennsteg über die Kanalenden hinausragt. Die Bohrungen für die Kanülen können unter einem Winkel $\alpha \leq 90°$ in die parallel zum Trennsteg verlaufenden Kanäle einmünden.

Die mit der Erfindung erreichten Vorteile sind insbesondere darin zu sehen, daß durch das Zusammenführen der Komponenten unmittelbar hinter dem Ende des Trennsteges ein schlanker Sprühkegel entsteht, der ein gleichmäßiges Vermischen der Komponenten und ein zielsicheres Besprühen der Objekte auch aus nächster Nähe bei niedrigem Sprühdruck ermöglicht. Durch den Trennsteg wird außerdem der vorzeitige Kontakt der unterschiedlichen Komponenten untereinander vermieden, was zu Verstopfungen der Kanäle führen würde. Der Sprühkopf eignet ich insbesondere zum Applizieren von chirurgischem Gewebeklebstoff, der aus den Komponenten in situ nach Passieren des Trennstegendes gebildet wird.

Im folgenden wird die Erfindung von lediglich einen Ausführungsweg zeigenden Zeichnungen näher erläutert. Es zeigen:

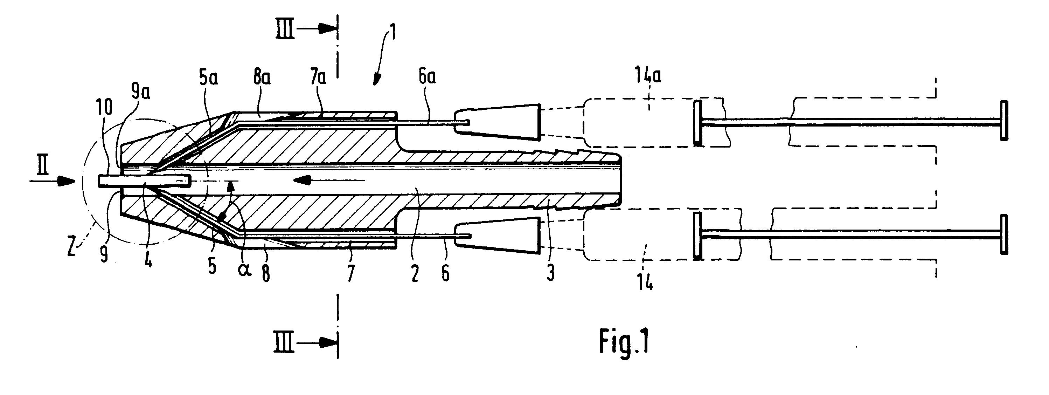

Figur 1 den Sprühkopf in Draufsicht geschnitten,

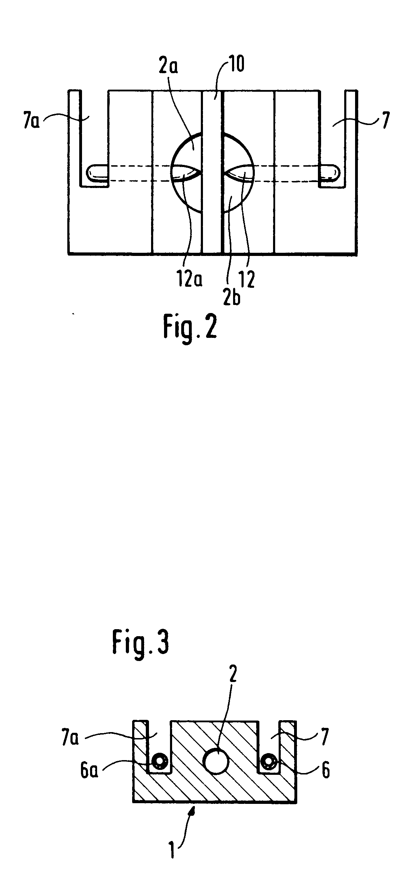

Figur 2 die Ansicht II von Figur 1,
Figur 3 den Schnitt III - III von Figur 1 und

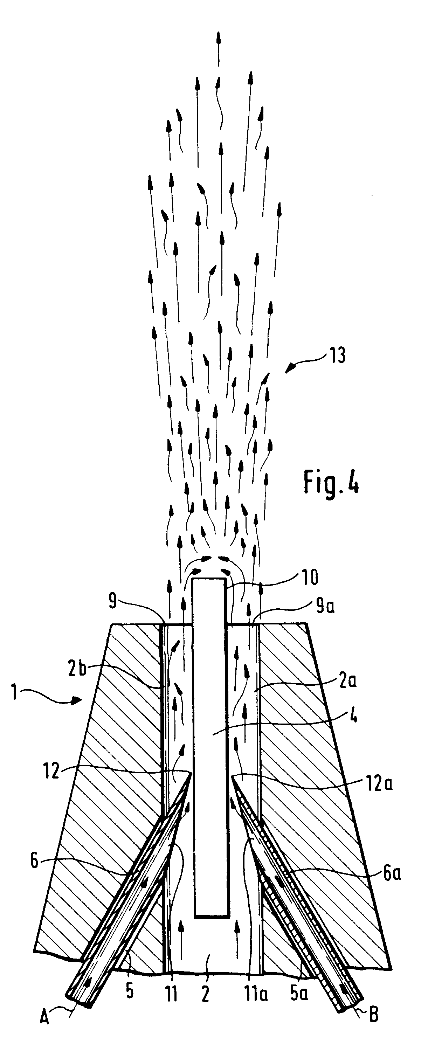

Figur 4 den Ausschnitt "Z" der Figur 1 mit Sprühbild in Draufsicht.

Der Sprühkopf 1 weist einen Gaskanal 2 mit Gasanschluß 3 auf. Im Gaskanal 2 ist ein Trennsteg 4 angeordnet, der den Gaskanal im mehrere parallel zum Trennsteg verlaufende Kanäle 2a, 2b aufteilt. In die Kanäle 2a, 2b münden Bohrungen 5, 5a zur Aufnahme von Kanülen 6, 6a. Zum Führen der Kanülen im Sprükopf gehen die offenen Enden der Bohrungen 5, 5a in Rinnen 7, 7a über, die parallel zum Gaskanal 2 im Sprühkopf 1 angeordnet sind. Um ein vorzeitiges Vermischen der Komponenten zu verhindern, ragt der Steg 4 über die Kanalenden 9, 9a hinaus. Die Ausnehmungen 8, 8a dienen dem Einführen der Kanülen in die Bohrungen. Nach dem Einführen werden die Kanülen in die Rinnen 7, 7a eingehängt und fixiert. Die Spitzen 12, 12a der Kanülen sollen an den Trennsteg 4 anstoßen und ihre abgeschrägten Öffnungen 11, 11a gegen die Strömungsrichtung des Gases weisen. Das aus dem Sprühkopf 1 herausragende Ende 10 des Trennsteges kann kantig oder abgerundet ausgeführt sein. Die Komponenten A und B werden über die Kanülen 6, 6a den Gaskanälen 2a, 2b zugeführt, vom Gas (Luft) mitgerissen und hinter dem Ende 10 des Trennsteges 4 verwirbelt, so daß sie reagieren können. Mit 13 ist das Sprühbild und mit 14, 14a sind die Einwegspritzen für die Komponenten A und B angedeutet. Besteht das Mehrkomponentenmaterial aus drei oder mehr Komponenten, kann der Trennsteg 4 in Querschnitt drei - oder vieleckig ausgebildet sein, so daß der Gaskanal 2 entsprechend der Anzahl der Komponenten aufgeteilt wird. In diese Kanäle münden dann ebenso viele Bohrungen mit Ausnehmungen und Rinnen.

## Patentansprüche

1. Sprühkopf zum Applizieren eines Mehrkomponentenmaterials mittels Gas, wobei der Sprühkopf (1) einen Gaskanal (2) mit Gasanschluß (3) aufweist, der durch einen Trennsteg (4) in mehrere parallel zum Trennsteg (4) verlaufende Kanäle (2a, 2b) aufgeteilt wird, in diese Kanäle (2a, 2b) Bohrungen (5, 5a) zur Aufnahme von Kanülen (6, 6a) münden die offenen Enden der Bohrungen (5, 5a) in Rinnen (7, 7a) zum Führen der Kanülen (6, 6a) übergehen, die parallel zum Gaskanal (2) im Sprühkopf (1) angeordnet sind, und der Trennsteg (4) über die Kanalenden (9, 9a) hinausragt.

## Claims

1. A spray head for the administration of a multi-component material by means of gas, the spray head (1) having a gas channel (2) with gas connection (3), which is divided by a separating web (4) into a plurality of channels (2a, 2b) running parallel to the separating web (4), there opening out into these channels (2a, 2b) boreholes (5, 5a) for receiving cannulas (6, 6a), the open ends of the boreholes (5, 5a) going into grooves (7, 7a) for guiding the cannulas (6, 6a) which are arranged parallel to the gas channel (2) in the spray head (1), and the separating web (4) protruding beyond the channel ends (9, 9a).

## Revendications

1. Tête de pulvérisation servant à appliquer, à l'aide d'un gaz, un matériau formé de plusieurs constituants, dans laquelle il est prévu un canal (2) d'adduction du gaz comportant un raccord à gaz (3) et qui est subdivisé, par une barrette de séparation (4), en plusieurs canaux (2a,2b) qui sont parallèles à la barrette de séparation (4) et dans lesquels débouchent des perçages (5, 5a) destinés à recevoir des canules (6, 6a), les extrémités ouvertes des passages (5, Sa) se prolongeant par des conduits (7, 7a) servant au guidage des canules (6, 6a) et qui sont parallèles au canal (2) d'adduction du gaz dans la tête de pulvérisation (1), la barrette de séparation (4) faisant saillie au-delà des extrémités (9a) des canaux.

Fig.1

EP 0 302 411 B1

Fig. 2

Fig. 3

Fig.4